# EUROPEAN PATENT APPLICATION

(11) **EP 2 960 653 A1**
(43) Date of publication of application: **30.12.2015**
(21) Application number: 14173757.7
(22) Date of filing: 24.06.2014
(51) Int. Cl.: G01N 33/68

(54) **Diagnostic kit and method for the identification of the manipulation of muscle mass in a domestic animal**

(71) Applicant: Stichting Kwaliteitsgarantie Vleeskalversector, 3706 AR Zeist (NL)
(72) Inventor: Van der Weg, Cornelis Peter Van, 3706 AR Zeist (NL)
(74) Representative: Algemeen Octrooi- en Merkenbureau B.V.

(57) **Abstract**

The invention relates to a diagnostic method and test kit for the identification of the manipulation of muscle growth in domestic animals such as cattle, wherein the animal is tested for at least one biomarker related to myostatin or myostatin-related pathways, wherein the levels of the at least one biomarker are compared with a control. The test reliably shows manipulation of the mystatin-related pathways based on several suitable biomarkers, in particular levels of anti-mostatin antibodies.

## Description

### Field of the invention

The invention relates to a diagnostic method and kit for the identification of the manipulation of muscle mass in a domestic animal.

### Background of the invention

Growth and differentiation factor-8 (GDF-8), also known as myostatin (MSTN), is a protein that regulates muscle mass in a number of species, and is present in both developing and adult muscles (Lee SJ, Mc Pherron AC. 2001. Regulation of myostatin activity and Muscle Growth. Proceedings of the National Academy of Sciences and the United States of America 98, 9306-9311). GDF-8 is a negative regulator of muscle mass. GDF-8 knock-out mice, as well as transgenic mice expressing myostatin inhibitors, showed an increased muscle growth. Apparently, myostatin inhibitors or inhibitors of the expression of the GDF-8 gene may lead to increased muscle growth.

There is a suspicion that the knowledge regarding the function of GDF-8 is applied in unapproved and uncertified methods to raise domestic animals intended for slaughter, which would result in unnaturally fast achievement of the desired weight for slaughter an/or improved yield of meat. In many jurisdictions, the use of such methods would be an illegal. However, there is currently no test available that can prove if such methods are used on a particular animal.

### Summary of the invention

It is an object of the invention to provide a diagnostic test that can provide an indication or proof of manipulation of muscle growth involving myostatin/GDF-8 related techniques, in domestic animals, in particular in domestic animals raised for meat production.

The invention provides a diagnostic methodology for the identification of the manipulation of muscle growth in a domestic animal, wherein the animal is tested for at least one biomarker related to myostatin or myostatin-related pathways, wherein the levels of the at least one biomarker are compared with a reference control. The biomarker comprises at least one protein, its coding mRNAs or one or more transcription factors involved in the regulation of myostatin levels. In an aspect of the invention, multiple biomarkers were found to be influenced when myostatin levels are manipulated, for instance through immunisation, for obtaining a faster and/or a more substantial muscular growth.

Changes in biomarkers related to myostatin provide an indication that myostatin levels are manipulated in an unnatural way. For a reference control, preferably data from an animal of the same species and breed is used, as myostatin levels may vary across certain breeds. Also the reference animals are preferably in a comparable stage of growth development. In addition, the reference animals are preferably of the same sex. More preferably, the reference animals are from the same geographical area, and have a comparable diet.

Myostatin and myostatin-related pathways are for instance described in: Han HQ, Mitch WE. "Targeting the myostatin signaling pathway to treat muscle wasting diseases." Curr Opin Support Palliat Care. 2011;5(4):334-41. doi: 10.1097/SPC.0b013e32834bddf9; Rodino-Klapac LR, Haidet AM, Kota J, Handy C, Kaspar BK, Mendell JR. "Inhibition of myostatin with emphasis on follistatin as a therapy for muscle disease." Muscle Nerve. 2009;39(3):283-96. doi: 10.1002/mus.21244.; Trendelenburg AU, Meyer A, Rohner D, Boyle J, Hatakeyama S, Glass DJ. "Myostatin reduces Akt/TORC1/p70S6K signaling, inhibiting myoblast differentiation and myotube size." American Journal of Physiology - Cell Physiology. 2009;296(6):C1258-C70.

In a preferred embodiment, the biomarker comprises at least one biomarker selected from the group consisting of Myostatin Propeptide, leptin, resistin, follistatin-like 1, follistatin, prolactin, insulin-like growth factor 1, Activin receptor IIB, members of de SMAD family of proteins and anti-Myostatin IgG antibody.

Testing for multiple biomarkers improves the reliability of the test. Preferably, the animal is tested for multiple biomarkers related to myostatin or myostatin-related pathways, wherein the multiple biomarkers are combined to form a fingerprint profile which is compared to a control fingerprint profile. The fingerprint control profile is preferably obtained from an animal of the same specie, sex and breed.

The invention provides a diagnostic method for the identification of the manipulation of muscle growth in a domestic animal by reducing the active amount of myostatin in the animal, wherein the comprises means for the quantification of anti-myostatin antibodies in at least part of the animal. Anti-myostatin antibodies inhibit the function of myostatin, which may lead to increased muscle growth. An increased amount of antimyostatin compared to untreated reference animals is indicative of intentional manipulation of muscle growth. The antibodies may for instance have been administered to the animals, or the anti-myostatin levels may have been raised by immunization with a suitable agent. If the observed animal is a mammal, the anti-myostatin antibodies may for instance be IgA, IgD, IgE, IgG or IgM, but IgG is considered the most suitable antibody type. In non-mammalian animals, other antibody types may be appropriate. The determination of the antibody levels would be performed in duplicate in order to provide the required statistical certainty.

In an embodiment of the invention, the effective amount of myostatin is reduced by immunization of the animal with at least one vaccine capable of inducing an anti-myostatin immunoreaction in the animal, preferably myostatin, a myostatin fragment or a myostatin analogon, causing an increased quantity of anti-myostatin antibodies in the animal compared to non-manipulated reference animals.

According to one aspect of the invention, the determined quantity of anti-myostatin antibodies is compared to at least one predetermined threshold, wherein the threshold is determined from quantities of anti-myostatin antibodies in a non-manipulated reference animal, and wherein, in an animal wherein the quantity of anti-myostatin antibodies is significantly above the threshold, the animal is diagnosed as positive, and wherein, in an animal wherein the quantity of anti-myostatin antibodies is essentially equal to or not significantly above the threshold, the animal is diagnosed as negative. The threshold may depend on the specific race and type of domestic animal. Reliable threshold levels can be determined based on collected measurement data, using statistical methods known in the art. Multiple threshold levels can be determined, for instance a threshold level above which the animal is determined to be conclusively positive and a level below which the animal is believed to be conclusively negative. Reliable conclusive levels may for instance be set at 95% or 99% statistical certainty.

In a case the quantity of anti-myostatin-binding antibodies is above the threshold, but not by a significant amount, the animal may be diagnosed as suspicious. Animal diagnosed as suspicious may be retested using the same diagnostic method. A retest may reveal pre-analytical or analytical errors or biological variation.

Preferably, the domestic animal is an animal raised for meat production. The animal is preferably selected from the group consisting of cattle, sheep, horse, goat, pigs, poultry, or fish. In a preferred embodiment, the domestic animal is cattle. Cattle includes various breeds of livestock, and mixed breeds, including well known breeds such as Friesian, Holstein, Limousin, Fries Hollands, Holstein Friesian, Roodbont, MRIJ (Meuse Rhine Yssel), Blaarkop, Blonde d'Aquitaine, Piemontese, and cross breeds thereof.

In a preferred embodiment, the diagnosed domestic animal is a young animal, preferably younger than one year old. As young animals are growing fast, the effect on muscle growth has a relatively large impact when compared to adolescent animals.

In a preferred embodiment, wherein the quantification of anti-myostatin antibodies is performed on at least one sample taken from the animal, wherein the quantity of anti-myostatin antibodies is determined for at least one of the sample sources selected from the group consisting of blood, plasma, serum, muscle tissue, skin and hair.

Multiple samples may be taken from the same sample source or different sample sources. Certain sample sources may be more efficient for obtaining certain types of antibody, for instance anti-myostatin IgG would be best determined in samples taken from blood serum. In a preferred embodiment, at least one sample is taken from blood serum.

In a preferred embodiment, the determination of anti-myostatin antibodies comprises the steps of:
- providing a solid surface, preferably a microtiter plate, comprising immobilized myostatin, myostatin fragment or a myostatin analogon, capable of binding the myostatin-binding antibodies to be quantified;
- Contacting a sample from the animal comprising anti-myostatin antibodies to the solid surface, such that the anti-myostatin antibodies to be quantified bind to the immobilized myostatin, myostatin fragment or myostatin analogon;
- Washing the solid surface; and
- Determining the amount of anti-myostatin antibodies bound to the solid surface.

Methods to immobilize peptides such as myostatin on a solid surface are known in the art, and may have a natural or synthetic origin. The sample is usually pretreated, for instance by dilution in a suitable buffer, prior to contacting the sample with the solid surface.

Washing is typically done by a buffer solution. By washing, non-binding sample matter, in particular antibodies not binding to myostatin are removed. The determination of the antibodies is typically done by chemically coupling a detectable label to the antibodies. Various suitable assays are known in the art, and the method may include an assay selected from the group consisting of an enzyme assay, a chromogenic assay, a luminescence assay, a fluorogenic assay, and a radio immune assay, and an immune-PCR assay.

In a preferred embodiment, the determination of the amount of anti-myostatin antibodies immobilized on the solid surface is established by coupling a detectable label directly and selectively binding to the anti-myostatin antibodies. The detectable label comprises an antibody specifically to antibodies produced by the species of domestic animal. For instance, if the tested animal would be cattle, the anti-myostatin antibodies produced by the cattle are directly and specifically bound by anti-cattle antibodies.

In another preferred embodiment, the antibodies in the sample are labelled with a crosslinking reagent, wherein the crosslinking reagent is reacted with the detectable label after binding with the immobilized myostatin, myostatin fragment or myostatin analogon. Various suitable crosslinking agents are known in the art, a preferred crosslinking agent is biotin.

In a preferred embodiment, the detectable reagent comprises a fluorescent label detectable by fluorescence quantification. Fluorescent labels give a reliable measurable signal that correlates to the number of bound anti-myostatin antibodies.

In another preferred embodiment, the detectable reagent comprises a DNA label detectable through immuno-PCR. The DNA is replicated by PCR and subsequently quantified. Immuno-PCR is a very sensitive method, particularly suitable for determining small amounts of detectable material.

The invention further relates to a diagnostic kit for performing a diagnostic method as described herein. In a preferred embodiment, the kit comprises at least a myostatin, myostatin fragment or myostatin analogon capable of selectively binding anti-myostatin antibodies, immobilized on a solid surface. In addition, the kit may comprise materials for taking and treating samples, buffers, positive and negative control samples, and a manual.

The term "solid phase" does not imply any specific limitations, and may include any insoluble polymer material, for polyamide or a vinyl polymer (e.g., poly(meth)acrylate, polystyrene and polyvinyl alcohol, or derivatives thereof), a natural polymer such as cellulose, dextran, agarose, chitin and polyamino acids, or an inorganic polymer, such as glass or metallohydroxide.

The myostatin, myostatin fragment or myostatin analogon can be immobilized on the solid phase directly by covalent coupling or via a carrier such as a linker molecule. Suitable methods to link peptides to solid surfaces are known in the art.

The solid surface may be provided in various forms. It is preferred if the solid surface is selected from the group consisting of plates, membranes, paper, film, strips, and pearls. Particularly suitable are microtiter plates having various numbers of wells, and which may be made of various materials.

In a preferred embodiment, the kit may further comprise a detectable reagent capable of binding to anti-myostatin antibodies bound to anti-myostatin antibodies, preferably either by direct binding or through a crosslinking reagent.

The following non-limiting examples will now further elucidate the invention.

### Example 1: Immunization experiment on calves

In order to validate the test method, calves animals were treated with a myostatin blocking treatment, leading to lower active myostatin levels, by immunization. Samples from the treated animals were compared with control animals belonging to the same breed.

### 1.1 Preparation of bovine test animals.

Animals were randomly assigned into treatment groups. Twenty one (21) Holstein-Friesian calves (12 male and 9 female) between 9 - 14 weeks of age were included in the study. 7 animals were used as a control group, whereas 14 animals were to be immunized in order to lower active myostatin levels. The control group was only injected with the injection fluids, whereas the second group was treated with a synthetic myostatin peptide. The treatments were administered intramuscularly on day 0, day 35 and day 88 of the treatment. Animals were monitored during 19 weeks from the start of the treatment.

The calves were 8 weeks old at the start of the experiment. Before and during the treatment, the calves were fed using equal and controlled amounts of commercially available CMR (Calf Milk Replacer) and Fibramix.

The preparation was aiming to raise anti-myostatin antibodies in the calves with the purpose of reducing levels of active myostatin. All calves were treated with a myostatin blocker three times with during the observation period. Physical data and samples were collected during the life-time of the calves, such as general health, local reactions, and body weight. Serum samples were collected. Some clinical signs were observed, but they remained within a range that was considered normal for a 19 weeks fattening period. The applied treatments did not impact the general health of the calves. The animals were healthy at the time of slaughter and all slaughter samples were collected.

### 1.2 Chemicals

The synthetic peptide, further referred to as myostatin peptide powder, derived from the myostatin polypeptide sequence, having the sequence CKMSPINMLYFNGKEQIIYG, was obtained from Genaxxon bioscience GmbH, Ulm, Germany, as the free acid with the N-terminus conjugated with a KLH group (keyhole limpet hemocyanin), purified by HPLC.

Myostatin peptide powder was dissolved in sterile arachis oil, and mixed with ViteSel, Freund's Adjuvant Incomplete, or Freund's Adjuvant Complete for injection. These materials are all commercially available. Vitesel is a commercially available vitamin injection fluid for calves containing alpha-tocoferolacetate and selenium. The control group was injected with the same injection fluid, but without the added peptide.

### 1.3 Sampling

Multiple blood samples were collected at weekly intervals and immediately stored at selected times during the treatment. At the end of the observation period at 19 weeks, all study animals were slaughtered in a licenced slaughterhouse, and tissue samples were taken.

### Preparation of Serum

Blood was allowed to clot at room temperature and serum was separated by centrifugation. Each serum sample was divided into aliquots and stored at -80°C+/- 5°C until further processing.

### 2. Testing for anti-myostatin antibodies

The collected blood serum from the calves in section was tested for anti-myostatin antibodies.

### 2.1 Materials and Reagents

The measurements were preformed on an Imperacer® Workstation (catalog number 25-001; Supplier: Chimera Biotec GmbH, Germany), using an Imperacer® Assay Development Kit (ADK; catalog number 20-007; Supplier: Chimera Biotec GmbH, Germany) which comprises a number of buffers. For detection, the Alternate Imperacer® Detection Conjugate "CHI-Bovine-IgG" (conjugated antibody: anti-Bovine IgG-heavy and light chain Antibody, Sheep Polyclonal, catalog number: A10-115; Supplier: Bethyl Laboratories, Inc., USA) was used. The Capture Reagent for binding anti-myostatin antibodies to the solid surface was GDF-8 (myostatin); (catalog number: 788-G8-010/CF; Supplier: R&D Systems, Germany). The used equipment, reagents and buffers could be replaced by other commercially available materials.

### 2.3 Analytical Assay Procedure

### Step 1: Plate preparation (I)

Microplates were coated with GDF-8 (myostatin) at: 1 µg/ml (30 µl/well), and incubated on the Imperacer® plate material for >12h at 4°C.

### Step 2: Plate preparation (II)

The coated microplate wells were coated with Imperacer® wash buffer "A" using washer-program "block", block against unspecific interaction with "chimera direct block" solution (240 µl/ well, 1 min/RT) and with Imperacer® wash buffer "B". After this treatment, the microplate wells were ready for sample incubation.

### Step 3: Sample Incubation

Serum samples were diluted with sample dilution buffer SDB3100 in low-bind vials and incubated for 1h at room temperature in GDF-8 coated wells, 30 µl/well in double determination, using orbital shaking (750 rpm).

### Step 4:Detection Conjugate

After incubation with sample, the micro plates were washed with Imperacer® wash buffer "B". Incubate wells with Imperacer® detection conjugate "CHI-Bovine-IgG" (1/30 dilution in Conjugate Dilution Buffer) for 30min at room temperature.

### Step 5: Preparation for PCR

The wells were subsequently washed with Imperacer® wash buffers "B" and "A". 30 µl/well of ready-to-use Imperacer® PCR-mastermix were added to each well. The wells were sealed using real-time PCR foil, and placed into the Imperacer® reader 50 cycles at 95°C with 12 sec per cycle, at 50°C for 30 sec and at 72°C for 30 sec.

The samples were analysed in duplicate following the analytical assay procedure described above, including negative and positive controls. All samples from a single subject were be run in one assay whenever possible; multiple subjects may be run in one assay.

### 2.5 Results

For assay evaluation, a blind and an open test were perfomed. All measured signals were normalized by subtraction of the average negative control signal.

Both in the blind and open tests, a clear association was observed between the levels of measured antimyostatin IgG antibodies and treatment with the synthetic peptide, compared to the negative control animals. The treated animals showed a significantly higher level of antimyostatin antibodies compared to the reference animals that had received the negative control preparation. An increase in antimyostatin antibodies was observed during the treatment, e.g. samples taken at a later time during the study showed higher anitimyostatin IgG levels.

Depending on selection of "Threshold Level", positive animals can be identified. "Prudent" threshold levels (e.g. "progressive") result in higher numbers of false-positive controls, whereas "higher" Threshold levels ensure only true positives, but will not identify all treated animals depending on response to treatment.

The analytical assay procedure response data were recorded as Imperacer® instrument response (ΔCt; delta-Ct). The sample status was determined using an analytical threshold value 237.1 ng/ml; below this threshold value the sample was judged to be negative, whereas values above this value were identified as possibly positive. Samples above the analytical threshold value of 382.1 ng/ml were determined as positive, whereas values between the two values were deemed to be suspicious.

Suspicious animals may need additional testing, for instance for additional biomarkers or histological markers (e.g. muscle fibre size diameter), to conclusively determine a positive or negative result. Combining various biomarkers and other parameters provides further certainty on the manipulation of muscle mass through alterations of the myostatin levels.

### 3. Biomarkers test

Mice were immunized with myostatin, and samples from the treated mice were subjected to multiple assays for various biomarkers related with the myostatin pathway.

### 3.1 treatment of mice

In order to assay biomarkers related to myostatin, adult male BALB/c mice were immunized using a synthetic myostatin peptide as used above. The synthetic peptide derived from myostatin, having the sequence CKMSPINMLYFNGKEQIIYG, was obtained from Genaxxon bioscience GmbH, Ulm, Germany, as the free acid with the N-terminus conjugated with a KLH group (keyhole limpet hemocyanin), purified by HPLC.

For negative control, a group untreated mice were used. Adult Mice treated with siRNA were used as a positive control. The synthetic peptide was applied by a single injection at a dose of 40 µg/ protein/Mouse in Freunds complete adjuvant (FCA). Application of siRNA was done via osmotic micro pumps for 28 days. Each group contained 10 mice. After 28 days, blood serum was isolated from the mice and subjected to the biomarker test as described below.

### 3.2 Biomarker assay

Microplates and reagents were obtained from Chimera Biotec GmbH, Germany: Microplate Modules (Item No. C-001), Microplate Module Frame (Item No. C-002), PCR-Foil, Storage-Foil, Buffer Reservoir (Item No. C-003), Coating Buffer (Item No. C-010); Wash-Buffer A (20x concentrate) (Item No. C-011), Wash-Buffer B (20x concentrate) * (Item No. C-012); Chimera Direct Block (Item No. C-013), AnySource® Sample Dilution Buffer: SDB6000 Basic (Item No.: tbd), Ready-to-use aliquots for 16 wells (2 microplate modules) each, Conjugate Dilution Buffer-b (CDB-b) (Item No. C-021), PCR-Mastermix (Item. No. C-022), Various, analyte-specific Imperacer® Detection Conjugates (e.g. "CHI-Leptin"). Various analyte-specific capture antibodies are commercially available.

Measurements were performed on an Imperacer® Workstation (Chimera Biotec GmbH, Germany; Cat. No. 25-001 or 25-002, using various digital pipettes, multichannel pipettes, a microplate compatible PCR-thermocycler, and a multichannel microplate washer, and other standard laboratory equipment. Commercially available protein low bind cups were used for minimization of unspecific binding of low antigen or reagent concentrations against the surface of the cups

### 3.3 Assay procedure

Wash buffers were brought to room temperature before use and diluted. Wash-Buffer A was diluted by adding 50 ml of Wash-Buffer A (20x concentrate) into 950 ml deionized and DNase-free water (e.g. autoclaved) to prepare 1000 ml of Wash-Buffer A. Wash-Buffer B was diluted by adding 100 ml of Wash-Buffer B (20x concentrate) into 1900 ml deionized and DNase-free water (e.g. autoclaved) to prepare 2000 ml of Wash-Buffer B. All aliquoted components were stored on ice during the assay procedure. All samples, standards and controls were performed in duplicate.

### (I) Immobilization of Capture Antibody

The analytical Target of the Capture Antibody (Antigen) were coated from stock concentrated solutions that are commercially available. The list below shows the concentrations of the coating solutions and dilution factors for coating. The stocks were diluted with coating buffer.
Goat anti-mouse 5µg/mlc = 1mg/ml 1 : 200
Leptin 10µg/mlc = 200µg/ml 1 : 20
IGF1 5µg/mlc = 1mg/ml 1 : 200
Adiponectin 5µg/mlc = 1mg/ml 1 : 200
FSTL15 µg/ml c = 1 mg/ml 1 : 200
Activin II B 5µg/mlc = 1mg/ml 1 : 200
Myostatin Propeptid 5µg/mlc = 1mg/ml 1 : 200
Prolactin 5µg/mlc = 1mg/ml 1 : 200
Myostatin (GDF8) 1µg/mlc = 100µg/ml 1 : 100 30µl/well of the capture antibody dilution was added into the corresponding microplate wells. The wells where sealed with storage foil and spun down by centrifugation. The incubation was overnight at 4°C. Capture-antibody coated plates can be stored at 4°C for at least 4 weeks.

### (II) Sample Preparation and Incubation

All samples and standards were perfomed in duplicate. The standards were prepared fresh in appropriate standard-curve range for every plate. The standards are prepared in low-binding 1.5 ml vials. Below lists the amount of standard and buffer solution.
a) Mouse IgG 1 µl + 499µl 1 + 99
b) Leptin 8µl + 72µl
c) IGF 1 2.6µl + 77.3µl
d) Adiponectin 1µl + 99µl 8 + 72
e) FSTL 1 1 µl + 99µl
f) Activin II B 20µl + 180µl
g) Myostatin Propeptid 20µl + 180µl
h) Prolactin 20µl + 180µl
i) GDF8 7µl + 133µl

The capture-antibody coated modules were washed with wash the program "BLOCK" (see below) in Imperacer® Washer. The washing steps were carried out using the Imperacer® Microplate Washer supplied the your Imperacer® Workstation (Cat. No. 25-001 or 25-002, Chimera Biotec, Germany).

240 µl/well of the Chimera Direct Block was added, and agitated for 30-60 seconds at room temperature with orbital shaking. After that, the wells were washed using the wash program "WASH" in Imperacer® Washer. 30µl of the diluted standards and samples were added to each well. The samples were incubated for 1 hour at room temperature and orbital shaking.

### (III) Imperacer® Conjugate Binding

The modules were washed after incubation using the wash program "Wash" in Imperacer® Washer. The appropriate Imperacer Conjugates were diluted in Conjugate Dilution Buffer "CDB" immediately prior to use, according to the dilution factors below.
Mouse IgG CHI Mouse 1 : 100
Leptin CHI Leptin 1 : 1000
IGF 1 CHI IGF 1 1 : 30
Adiponectin CHI Adiponectin 1 : 300
FSTL 1 CHI FSTL 1 1 : 100
Activin II B Act II B 1 : 30
Myostatin Propeptid CHI Myostatin 1 : 300
Prolactin CHI Prolactin 1 : 300

### GDF8

The conjugates were incubated with 30 µl/well of the diluted Imperacer® Conjugate solutions for 45 min at room temperature / orbital shaking.

### (IV) Signal read-out by real-time PCR

The wells in the modules were washed with the wash program "PCR" in the Imperacer® Washer. Modules were removed from the microplate module frame and placed in a 0.2 ml 96-well rack. 30 µl of the PCR-Mastermix were added into each well and the wells were sealed and subsequently transferred to the Imperacer® PCR instrument, using an Optical Cover Compression Pad on the sealed modules. Subsequently, the PCR program was started.

PCR was performed using an automated program, selecting FAM (emission at 518 nm) as fluorophor, and at 50°C in the PCR-cycle, using the following steps and repeats:
4 min 95°C 1x
12 sec 95°C 50x
30 sec 50°C
30 sec72°C

### 3.4 Results

After the treatment of the mice, it was found that all mice treated with the myostatin synthetic peptide showed significant increase in body weight. In treated mice, a distinctive deviation between the read-out signal for the indicated biomarkers was This indicates that the indicated biomarkers Myostatin Propeptide, leptin, resistin, follistatin-like 1, follistatin, prolactin, insulin-like growth factor 1, Activin receptor IIB, members of de SMAD family of proteins and anti-Myostatin IgG antibody, can be used as indicators for the manipulation of myostatin pathways. Multiple of such biomarkers can be combined as a finger print profile. Comparing fingerprint profiles rather than single biomarkers increases the certainty of proof that myostatin pathways are indeed manipulated.

## Claims

1. Diagnostic method for the identification of the manipulation of muscle growth in a domestic animal, wherein the animal is tested for at least one biomarker related to myostatin or myostatin-related pathways, wherein the levels of the at least one biomarker are compared with a control.

2. Diagnostic method according to claim 1, wherein the biomarker comprises at least one biomarker selected from the group consisting of Myostatin Propeptide, leptin, resistin, follistatin-like 1, follistatin, prolactin, insulin-like growth factor 1, Activin receptor IIB, members of de SMAD family of proteins and anti-Myostatin IgG antibody.

3. Diagnostic method according to claim 1 or 2, wherein the animal is tested for multiple biomarkers related to myostatin or myostatin-related pathways, wherein the multiple biomarkers are combined to form a fingerprint profile which is compared to a control fingerprint profile.

4. Diagnostic method according to any of the preceding claims for the identification of the manipulation of muscle growth in a domestic animal by reducing the active amount of myostatin in the animal, wherein the method comprises means for the quantification of anti-myostatin antibodies, preferably anti-Myostatin IgG antibody.

5. Diagnostic method according to claim 4, wherein the effective amount of myostatin is reduced by immunization of the animal with at least one vaccine, preferably myostatin, a myostatin fragment or a myostatin analogon, capable of inducing an anti-myostatin immunoreaction in the animal, causing an increased quantity of anti-myostatin antibodies in the animal compared to non-manipulated reference animals.

6. Diagnostic method according to claim 4 or 5, wherein the determined quantity of anti-myostatin antibodies is compared to at least one predetermined threshold, wherein the threshold is determined from quantities of anti-myostatin antibodies in a non-manipulated reference animal, and wherein, in an animal wherein the quantity of anti-myostatin antibodies is significantly above the threshold, the animal is diagnosed as positive, and wherein, in an animal wherein the quantity of anti-myostatin antibodies is essentially equal to or not significantly above the threshold, the animal is diagnosed as negative.

7. Diagnostic method according to any of the preceding claims, wherein the domestic animal is an animal raised for meat production.

8. Diagnostic method according to any of the preceding claims, wherein the domestic animal is an animal selected from the group consisting of cattle, sheep, horse, goat, pigs, poultry, or fish, preferably cattle.

9. Diagnostic method according to any of the preceding claims, wherein the domestic animal is a young animal, preferably younger than one year old.

10. Diagnostic method according to any of the preceding claims, wherein at least one sample taken from the animal, wherein the quantity of anti-myostatin antibodies is determined for at least one of the sample sources selected from the group consisting of blood plasma, blood serum, muscle tissue, and hair, preferably blood serum.

11. Diagnostic method according to any of the preceding claims, wherein the biomarker is anti-myostatin antibodies, wherein the method comprises the steps of:
- providing a solid surface, preferably a microtiter plate, comprising immobilized myostatin, myostatin fragment or a myostatin analogon, capable of binding the myostatin-binding antibodies to be quantified;
- Contacting a sample from the animal comprising anti-myostatin antibodies to the solid surface, such that the anti-myostatin antibodies to be quantified bind to the immobilized myostatin, myostatin fragment or myostatin analogon;
- Washing the solid surface; and
- determining the amount of anti-myostatin antibodies bound to the solid surface, preferably by coupling a detectable label directly and selectively binding to the anti-myostatin antibodies.

12. Diagnostic method according to claim 14, wherein the determining of the amount of anti-myostatin antibodies is done using an antibody specifically binding to antibodies produced by the species of domestic animal.

13. Diagnostic method according to claim 14, wherein the antibodies in the sample are labelled with a crosslinking reagent, preferably biotin, wherein the crosslinking reagent is reacted with the detectable label after binding with the immobilized myostatin, myostatin fragment or myostatin analogon, preferably a fluorescent label detectable by fluorescence quantification or a DNA label detectable through immuno-PCR.

14. Diagnostic kit for performing a diagnostic method according to any of the preceding claims, wherein the kit comprises at least a myostatin, myostatin fragment or myostatin analogon capable of selectively binding anti-myostatin antibodies, immobilized on a solid surface.

15. Diagnostic kit according to claim 14, wherein the kit further comprising a detectable reagent capable of binding to anti-myostatin antibodies bound to anti-myostatin antibodies, preferably either by direct binding or through a crosslinking reagent.
